(19) Europäisches Patentamt
European Patent Office
Office européen des brevets

(11) **EP 2 473 465 B1**

(12) **FASCICULE DE BREVET EUROPEEN**

(45) Date de publication et mention de la délivrance du brevet:
**28.06.2017 Bulletin 2017/26**

(21) Numéro de dépôt: **10763795.1**

(22) Date de dépôt: **01.09.2010**

(51) Int Cl.:
*C07C 29/78* *(2006.01)*        *C07C 31/26* *(2006.01)*

(86) Numéro de dépôt international:
**PCT/FR2010/051822**

(87) Numéro de publication internationale:
**WO 2011/027078 (10.03.2011 Gazette 2011/10)**

(54) **POUDRE DE CRISTAUX DE MANNITOL DEFINE, SON PROCEDE DE FABRICATION**

MANNITOLKRISTALLPULVER MIT GERINGEM FEINPARTIKELANTEIL SOWIE VERFAHREN ZU SEINER HERSTELLUNG

MANNITOL CRYSTAL POWDER HAVING A LOW FINE-PARTICLE CONTENT, AND METHOD FOR PRODUCING SAME

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **01.09.2009 FR 0955959**

(43) Date de publication de la demande:
**11.07.2012 Bulletin 2012/28**

(73) Titulaire: **ROQUETTE FRERES**
**62136 Lestrem (FR)**

(72) Inventeurs:
• **BOIT, Baptiste**
**F-62400 Bethune (FR)**
• **LEFEVRE, Philippe**
**F-59660 Haverskerque (FR)**
• **LIS, José**
**F-59253 La Gorgue (FR)**

(74) Mandataire: **Cabinet Plasseraud**
**66 rue de la Chaussée d'Antin**
**75440 Paris Cedex 09 (FR)**

(56) Documents cités:
**EP-A1- 0 645 096     EP-A1- 1 138 661**

Remarques:
Le dossier contient des informations techniques présentées postérieurement au dépôt de la demande et ne figurant pas dans le présent fascicule.

EP 2 473 465 B1

**Description**

La présente invention est relative à une composition pulvérulente de cristaux de mannitol pauvre en fines particules. L'invention porte également sur le procédé d'obtention d'une telle composition pulvérulente de mannitol.

Le 1,2,3,4,5,6-hexanehexol ($C_6H_{14}O_6$), appelé communément mannitol, est un polyol obtenu par extraction de végétaux tels que des algues ou par synthèse telle que par hydrogénation catalytique du mannose ou du fructose. L'hydrogénation du mannose s'effectue avec un rendement stoechiométrique et fournit le mannitol. Alors que l'hydrogénation catalytique du fructose, bien que permettant l'obtention de mannitol, possède un rendement non stoechiométrique puisque l'hydrogénation du fructose n'est pas stéréospécifique et fournit du sorbitol en quantité égale.

Le mannitol présente un grand intérêt en raison de sa stabilité chimique, d'une hygroscopicité presque nulle, de son faible pouvoir calorique et de son index glycémique inférieur à celui du saccharose, tout en possédant avantageusement une solubilité élevée, un goût sucré et rafraichissant en bouche. De plus, le mannitol possède la particularité de n'être pas cariogène, ce qui présente un intérêt dans l'industrie pharmaceutique ou alimentaire, notamment dans le domaine des confiseries, et plus particulièrement des chewing-gums. Il permet donc la fabrication de confiseries non cariogènes dans la mesure où les autres ingrédients de la formulation n'apportent pas de sucres fermentescibles.

L'art antérieur décrit l'utilisation de mannitol cristallisé dans les procédés d'obtention de chewing-gums, qui comportent de manière générale cinq étapes (Formulation and production of chewing and bubble gum, édité par Douglas Fritz, Kennedy's Publications Ltd, London, UK). Au cours de la première étape, les différents composés sont mélangés au moyen d'un pétrin comprenant 2 pales en forme de z. Le cycle entier de l'opération dure de 15 à 20 minutes et les ingrédients sont ajoutés au fur et à mesure dans le pétrin. Afin de rendre la gomme base malléable, celle-ci est chauffée préalablement et en cours de mélange. En fin de malaxage, la température de la pâte est d'environ 50°C. Parmi les ingrédients constitutifs des chewing-gums, on distingue deux grands groupes que sont les éléments insolubles dans l'eau et donc dans la salive, tels que principalement la gomme base, et les éléments solubles dans l'eau conférant au chewing-gum sa saveur, tels que les édulcorants notamment. L'étape de mélange est suivie d'une seconde étape d'extrusion à chaud afin d'obtenir une bande de chewing-gum plus ou moins large suivant l'appareil utilisé. Afin de réduire l'épaisseur de la bande obtenue, une étape de laminage est prévue. Au cours de cette étape, la bande passe successivement entre plusieurs paires de rouleaux d'écartement décroissant. L'étape de laminage est suivie d'une dernière étape de formage/découpage, pouvant être une simple étape de mise en forme associée à une découpe ou prédécoupe de la bande obtenue avant conditionnement. Or, après l'étape d'extrusion à chaud, la bande de gomme est extrêmement collante. De fait, afin d'éviter sa destruction ou la perte de son intégrité au cours du laminage, il est classiquement procédé à une étape de saupoudrage sur les deux faces de la bande entre les étapes d'extrusion et de laminage. De nombreux agents sont utilisés dans les poudres de saupoudrage. On retrouve ainsi des agents fluidifiants ou des agents anti-mottants tels que le talc, le carbonate de calcium, le phosphate tricalcique, la silice ou les silicates. Tous ces agents minéraux sont susceptibles de dégrader les propriétés organoleptiques des chewing-gums obtenus. En effet, ces agents sont insolubles, sans saveur, voire désagréables en bouche.

Par ailleurs, la poudre la plus largement utilisée pour le saupoudrage est le talc. Or, le talc peut faire l'objet d'une contamination par un produit de nature chimique très proche et pourtant très toxique : l'amiante. Ainsi, le talc contaminé aurait été impliqué dans des processus de cancérisation, que ce soit du tube digestif suite à une absorption par voie orale, ou du tissu pulmonaire lors d'une absorption par voie respiratoire, notamment au cours de sa manipulation. La manipulation du talc est donc réglementée et des équipements de protection respiratoire sont obligatoires pour le personnel de production.

Le document EP 0 645 096 A1 et plus particulièrement son exemple 1 porte sur une poudre de mannitol obtenue par solubilisation de cristaux de mannitol, atomisation de la solution obtenue et enfin granulation de poudre obtenue. La poudre pulvérulente selon ce document EP 0 645 096 se distingue de l'invention en ce qu'elle n'est pas une composition pulvérulente de cristaux de mannitol directement obtenue par cristallisation d'une solution de mannitol.

Le document EP 1 138 661 A1 et plus particulièrement son exemple 1 porte sur un procédé d'obtention de granulats de cristaux de mannitol. La composition pulvérulente du document EP 1 138 661 n'est donc pas directement obtenue par cristallisation d'une solution de mannitol mais par granulation de cristaux de mannitol.

Afin de réduire les quantités de talcs incorporées au cours de l'élaboration des chewing-gums, des poudres de polyols non hygroscopiques, tels que notamment le Mannitol, sont régulièrement utilisés. Généralement, les poudres de polyols sont de très fine granulométrie afin de remplacer le talc, lui-même de granulométrie très fine (poudre de granulométrie inférieure à 40$\mu$m et de diamètre moyen inférieur à 10$\mu$m), et afin de ne pas être ressentie sur la langue lors de la dégustation du chewing-gum.

Cependant, un remplacement total du talc par ces poudres n'est pas recommandé puisqu'elles ont un très mauvais écoulement, les rendant inaptes au saupoudrage. Dans le cas d'un remplacement partiel, le talc ayant un bon écoulement, confère au mélange un écoulement toujours médiocre mais suffisant pour permettre le saupoudrage de la bande de chewing-gum. Or, même dans le cadre d'un remplacement partiel du talc, la réduction d'écoulement du mélange de poudre est telle qu'elle contraint au dépôt d'une grande quantité de poudre sur la bande de chewing-gum, induisant de fait un gaspillage important, une dégradation de la qualité des chewing-gums obtenus, ou une modification des conditions de réglage des appareils.

Par ailleurs, la faible granulométrie de ces poudres augmente la genèse de poussières en suspension dans l'air, accentuant ainsi les risques associés à la présence d'amiante dans le talc pour les manipulateurs.

Enfin, un phénomène de prise en masse des poudres de polyols de fine granulométrie dans leur conditionnement est observé. En effet, ces poudres sont instables en ce qu'elles mottent au stockage ou au cours du transport. Les masses obtenues ne peuvent être désolidarisées que sous l'exercice de forces très importantes.

Bien que l'utilisation d'anti-mottants dans l'alimentation engendre des contraintes réglementaires puisqu'ils peuvent être considérés comme toxiques ou dangereux, cette solution a été envisagée. Cependant, alors qu'une réduction du mottage a été mise en évidence dans le cas des poudres de polyols hygroscopiques, aucun changement de comportement similaire n'est observé pour des poudres de polyols peu ou non hygroscopiques. En conséquence, ces anti-mottants n'ont qu'un effet sur certaines poudres et pas d'autres tel est le cas du mannitol.

L'état de la technique propose également afin d'améliorer les caractéristiques des compositions pulvérulentes de mannitol d'effectuer après la cristallisation du mannitol, des étapes supplémentaires d'atomisation ou de granulation. Or, ces procédés s'avèrent relativement couteux.

Afin d'obtenir une poudre stable au stockage et au transport, ayant de bonnes caractéristiques d'écoulement, et particulièrement avantageuse lors de sa mise en oeuvre au cours des procédés d'obtention du chewing-gum, l'invention porte sur une composition pulvérulente de cristaux de mannitol obtenue par (i) cristallisation de mannitol dans un solvant, préférentiellement dans l'eau suivie (ii) d'une étape de séparation des cristaux de la suspension de cristaux obtenue (iii) d'une étape de séchage des cristaux et (iv) d'une étape de sélection, ladite composition présentant une répartition granulométrique en volume, déterminée par granulométrie laser, présentant :

- de 72 à 99,9%, de préférence de 75 à 99%, plus préférentiellement de 80 à 98%, et plus préférentiellement encore de 85 à 97% de particules de granulométrie supérieure à 75$\mu$m,

- de 0,1 à 60%, de préférence de 1 à 55%, plus préférentiellement de 2 à 40%, et plus préférentiellement encore de 3 à 35% de particules supérieure à 250$\mu$m,

- un diamètre moyen de 100 à 300$\mu$m, de préférence de 120 à 270$\mu$m, plus préférentiellement de 150 à 250 $\mu$m, encore plus préférentiellement de 170 à 230$\mu$m.

Au sens de l'invention, on entend par « composition pulvérulente de cristaux de mannitol » ou par « poudre de cristaux de mannitol », le produit obtenu par cristallisation d'une solution pure ou impure de mannitol. À savoir une solution comprenant de 20% à 100% sur matière sèches de mannitol. On citera en exemple le brevet européen EP0202168.

Un tel procédé a la particularité de permettre l'obtention d'une composition de cristaux majoritairement individualisés. Par « majoritairement » on entend plus de 50% de cristaux individualisés, préférentiellement de 60 à 100% voire de 70 à 99,9% et plus préférentiellement 80 à 98% de cristaux individualisées. Par l'usage du terme « individualisé » on entend la notion d'unité par opposition à un agglomérat, un agrégat ou un massé de cristaux.

Au sens de l'invention, on entend par « cristaux » des polyèdres de structure périodique, directement obtenus par cristallisation à savoir, par le passage d'un corps de l'état liquide, solvaté (dissous dans un solvant) ou gazeux à l'état solide.

Ainsi, une poudre de mannitol obtenue par granulation d'une composition cristalline ne constitue pas une poudre de cristaux de mannitol au sens de l'invention, en ce que les cristaux de cette poudre sont agglomérés. L'homme du métier parle d'agglomérats de cristaux. De même, une poudre obtenue par atomisation d'un sirop de mannitol ne constitue pas une composition de cristaux de mannitol au sens de l'invention car cette poudre est constituée de sphères ayant une structure en éponge composée de microcristaux associés en une masse avec ou sans phase amorphe. Chaque sphère étant obtenue par le séchage d'une goutte du sirop pulvérisé au cours de l'atomisation. L'homme du métier parle d'atomisat. Enfin, une composition de cristaux selon l'invention se distingue des massés obtenus par fondu en ce que les particules obtenues sont formées de microcristaux massés les uns sur les autres. Ces compositions (granulats atomisat et massés) sont différentes structurellement de la composition selon l'invention et de fait, ont des caractéristiques physiques et des applications distinctes.

Selon une première variante, la cristallisation s'effectue par des procédés de refroidissement ou d'évaporation d'une solution de polyol.

Selon une seconde variante, la cristallisation s'effectue par des procédés physicochimiques. Typiquement, la cristallisation s'effectue par adjonction d'un diluant, plus particulièrement d'un solvant organique tel qu'un alcool.

Selon une troisième variante, la cristallisation s'effectue de façon fractionnée c'est à dire par cristallisations successives, les cristaux obtenus à chaque étape sont solubilisés dans un solvant puis cristallisés à nouveau.

Typiquement, l'étape de cristallisation est suivie d'une étape de sélection des particules éventuellement précédée d'un broyage des cristaux obtenus.

Préférentiellement, la sélection de particules est effectuée par un procédé de classification par tamisage ou sur un séparateur pneumatique.

Les valeurs de répartition granulométrique sont déterminées sur un granulomètre à diffraction LASER type LS 230 de la société BECKMAN-COULTER, équipé de son module de dispersion poudre par aspiration (aspirateur de 1400 watts) de l'échantillon (voie sèche), en suivant le manuel technique et les spécifications du constructeur.

Les conditions opératoires de vitesse de vis sous trémie et d'intensité de vibration de la goulotte de dispersion sont déterminées de manière à ce que la concentration optique soit comprise entre 4 % et 12 %, idéalement 8 %.

La gamme de mesure du granulomètre à diffraction LASER type LS 230 est de 0,4 $\mu$m à 2.000 $\mu$m. Les résultats sont calculés en pourcentage volumique, et exprimés en micron ($\mu$m). Le mode de calcul utilisé est celui selon la théorie de FRAUNHOFER. Ainsi, une granulométrie supérieure à 75$\mu$m correspond à des particules mesurées entre 75 à 2000$\mu$m, et une granulométrie supérieure à 250$\mu$m correspond à des particules mesurées entre 250 et 2000$\mu$m.

La courbe de distribution granulométrique permet également de déterminer la valeur du diamètre moyen volumique (moyenne arithmétique) D4,3.

Selon une variante de l'invention, la composition pulvérulente de cristaux de mannitol comporte une note d'écoulement supérieure ou égale à 55, préférentiellement située entre 60 et 90, et plus préférentiellement située entre 65 et 85 encore plus particulièrement entre 68 et 80.

L'aptitude à l'écoulement est évaluée en utilisant l'appareil POWDER TESTER de type PTE commercialisé par la société HOSOKAWA. Cet appareil permet de mesurer, dans des conditions standardisées et reproductibles, l'aptitude à l'écoulement d'une poudre et de calculer une note d'écoulement, encore appelée indice de coulabilité, en se basant sur les travaux de M. Ralph Carr (1965). La note d'écoulement est calculée à partir des valeurs obtenues par la mise en oeuvre des quatre tests suivants : compressibilité, angle de repos, angle de spatule, Uniformité (voir manuel technique de l'appareil POWDER TESTER de type PTE). Pour ce dernier test, la granulométrie utilisée est celle obtenue par granulométrie laser décrite plus haut.

Une telle valeur d'écoulement confère à la composition pulvérulente selon l'invention de bonnes caractéristiques pour une utilisation au cours du procédé d'obtention de chewing-gum, notamment au cours duquel un réglage précis de la quantité de poudre à déposer sur la gomme base est possible. Cet écoulement est aussi un avantage pour les nombreuses utilisations du mannitol dans de domaine des médicaments. Il permet un remplissage plus facile des sachets, des gélules, et facilite aussi la production des comprimés autorisant un remplissage plus aisé des matrices des presses à comprimer.

Selon une variante avantageuse, la composition pulvérulente de cristaux de mannitol présente une richesse en mannitol de 96 à 100% en poids, de préférence entre 97% et 99,9% en poids, plus préférentiellement entre 98 et 99,8 % en poids.

Selon une autre variante avantageuse, la poudre de cristaux de mannitol présente une compressibilité comprise entre 30 et 15%, de préférence entre 27 et 12 %, et plus préférentiellement entre 24 et 10%.

Une telle valeur de compressibilité confère à la poudre de mannitol une meilleure stabilité de son état pulvérulent au stockage. Lorsque la valeur de compressibilité est supérieure à 20 %, la poudre ne présente pas d'écoulement libre et a tendance à former des voûtes dans la trémie (livret appareil PTE d'HOSOKAWA). Pour des valeurs particulières de compressibilité de 40 - 50 %, il devient même impossible de décharger le matériel de la trémie une fois que le matériel y a été stocké.

Avantageusement, la composition pulvérulente de cristaux de mannitol présente une densité aérée supérieure à 0,480 g/ml, de préférence comprise entre 0,540 et 0,700 g/ml, plus préférentiellement comprise entre 0,580 et 0,650 g/ml, et une densité tassée comprise entre 0,700 et 0,860 g/ml, de préférence comprise entre 0,725 et 0,830 g/ml, et plus préférentiellement comprise entre 0,750 et 0,800 g/ml.

Une telle valeur de densité aérée et de densité tassée confère à la poudre de mannitol une densité suffisamment élevée pour que les coûts de conditionnement et de transport respectent les standards du commerce, c'est-à-dire que les industriels ne soient pas obligés de conditionner et de transporter beaucoup d'air en plus du produit. Au contraire, des poudres de densité trop élevée présentent des défauts lors de leurs utilisations technologiques : manque de comprimabilité, dissolution lente, difficulté de dosage précis.

Les valeurs de densité tassée et aérée, et de compressibilité, de la composition pulvérulente de cristaux de mannitol selon l'invention sont déterminées en utilisant l'appareil POWDER TESTER type PTE commercialisé par la société HOSOKAWA, en suivant les spécifications du constructeur.

Cet appareil permet de mesurer, dans des conditions standardisées et reproductibles, l'aptitude à l'écoulement d'une poudre en mesurant notamment la densité aérée vrac et la densité tassée vrac et ensuite de calculer, à partir de ces données, les valeurs de compressibilité par la formule suivante :

$$\text{Compressibilité(\%)} = \frac{(\text{densité tassée} - \text{densité aérée})}{\text{Densité tassée}} \times 100$$

Les mesures de densité tassée et de densité aérée, sont réalisées sur l'appareil POWDER TESTER de type PTE, comme mentionné ci-avant, selon la méthode préconisée dans le mode d'emploi dudit POWDER TESTER (réglage par défaut sur 180 secousses pour la mesure de la densité tassée).

De telles compositions pulvérulentes ont une résistance au mottage particulièrement importante et ont de très bonnes caractéristiques d'écoulement, de densité vis-à-vis des mannitols de l'état de la technique et sont particulièrement adaptées pour une utilisation dans les procédés de fabrication de chewing-gums, du fait de leur bon écoulement et de leur faible tendance à créer des poussières

L'invention porte également sur le procédé d'obtention d'une composition pulvérulente de cristaux de mannitol comprenant de 72 à 99,9%, de préférence de 75 à 99%, plus préférentiellement de 80 à 98%, et plus préférentiellement encore de 85 à 97% de particules de granulométrie supérieure à $75\mu$m selon l'invention comprenant :

a) une étape de cristallisation de mannitol dans un solvant, préférentiellement dans l'eau,

b) une étape de séparation des cristaux de la suspension de cristaux obtenue,

c) une étape de séchage des cristaux,

d) une étape de sélection des cristaux, et

e) une étape de récupération de la composition pulvérulente.

Le séchage peut s'effectuer par des moyens connus de l'homme du métier tels que des lits d'air fluidisé par exemple, un séchoir pneumatique ou rotatif.

La séparation des cristaux de la suspension de cristaux obtenue s'effectue typiquement par une étape de centrifugation ou de filtration. La mise en oeuvre de telles méthodes de centrifugation ou de filtration étant connue de l'homme du métier

De façon avantageuse, l'étape de sélection b) s'effectue par un procédé de classification par tamisage ou pneumatique.

Par « sélecteurs pneumatiques », on entend des appareils séparant les poudres selon leur granulométrie par l'usage de flux d'air. De tels sélecteurs sont décrits dans l'article « Classification pneumatique » de Pierre BLAZY et El-Aïd JDID Technique de l'ingénieur, traité Génie des Procédés. Ces sélecteurs peuvent être à chambres de sélection statiques utilisant un courant gazeux horizontal ou vertical ou mixte, de tels sélecteurs peuvent être avec ou sans chicanes. Un autre type de sélecteur pneumatique est le sélecteur utilisant la force centrifuge. Parmi ces derniers, on décrit des cyclones statiques, les sélecteurs à rotor à axe horizontal et les sélecteurs mécanique à axe vertical.

De façon préférentielle, les poudres cristallines sont obtenues par cristallisation puis sélection de particules. Préférentiellement la sélection de particules est effectuée par un sélecteur pneumatique. Avantageusement le sélecteur pneumatique est un sélecteur statique préférentiellement à courant gazeux vertical. De façon particulièrement avantageuse, le sélecteur pneumatique est un sélecteur zig-zag.

Un tel sélecteur est décrit dans le brevet US 1.861.248.

La sélection dans un sélecteur Zig-Zag est une sélection gravitaire par air. Il s'agit d'un procédé de sélection dans lequel les particules solides sont classifiées selon leur comportement lors de leur chute, car dans la zone de sélection, elles sont soumises à la force gravitaire et à la force de traînée du flux d'air. La sélection est en fait basée sur la différence de trajectoires de particules non identiques dans la zone de sélection.

De façon avantageuse, le sélecteur zig-zag comporte plusieurs étages, le sélecteur comprenant préférentiellement 13 étages. La présence de ces étages permet d'utiliser le même air pour tous les étages, et de répéter la sélection à la fois dans le courant ascendant des particules légères et dans le courant d'air descendant des grosses particules.

Selon un mode de réalisation préféré du procédé conforme à l'invention, l'étape de sélection b) des particules s'effectue par un sélecteur zig-zag comportant un seul canal. Préférentiellement, le canal présente une largeur de 20 mm et une profondeur de 220mm.

Le sélecteur est construit en assemblant un nombre de sections ensemble avec un angle fixé afin de créer le canal du Zig-Zag. Préférentiellement, le sélecteur zig-zag présente des angles de 120°. Le canal a une section rectangulaire. Sa géométrie particulière et la direction du flux d'air induit alors deux courants de particules distincts : un courant de particules fines emportées par le courant d'air ascendant; un courant de particules grosses descendant le long de la paroi la plus basse de chaque section.

A chaque étage, les particules des deux courants sont donc soumises à une nouvelle sélection. Après quoi les particules continuent leurs mouvements dans le courant de particules originales ou sont transportées dans le courant de direction opposée.

La performance du sélecteur est déterminée par le comportement des particules à chaque étage d'une part et par l'interaction entre les étages d'autre part.

L'avantage du procédé conforme à l'invention est que le sélecteur Zig-Zag tel que décrit permet de classifier une composition pulvérulente de cristaux de mannitol en deux fractions (fines et grosses).

Pour ce faire, un jet d'air ascendant (air primaire) est envoyé dans le sélecteur Zig-Zag, sa vitesse permettant de caractériser le diamètre de coupure.

Les particules de diamètre supérieur au diamètre de coupure descendent malgré le jet d'air, alors que les autres sont entraînées par l'air ascendant.

De façon avantageuse, chaque étage du sélecteur zig-zag présente une hauteur de 92 mm. Préférentiellement, l'alimentation s'effectue au niveau du 9eme étage.

Selon une variante préférentielle, l'étape de sélection b) comprend les étapes suivantes :

b.1) alimenter un sélecteur Zig-Zag avec une composition pulvérulente de cristaux de mannitol présentant un diamètre moyen arithmétique compris entre 80 et 145 $\mu$m,

b.2) régler le débit d'air primaire de manière à récupérer une fraction de composition pulvérulente de cristaux de mannitol présentant un diamètre arithmétique compris entre 100 à 300$\mu$m, de préférence de 120 à 270$\mu$m, plus préférentiellement de 150 à 250 $\mu$m, et encore plus préférentiellement de 170 à 230$\mu$m, et une répartition granulométrie en volume, déterminée par granulométrie laser de 0,1 à 60%, de préférence de 1 à 55%, plus préférentiellement de 1 à 40%, et plus préférentiellement encore de 1 à 35% de particules supérieure à 250$\mu$m.

Avantageusement, le débit d'alimentation du sélecteur zig-zag en composition pulvérulente de cristaux de mannitol selon l'étape b.1) est de 9 à 15Kg/h, préférentiellement 10 à 13Kg/h, et plus préférentiellement 12Kg/h.

La composition pulvérulente de cristaux de mannitol conforme à l'invention peut avantageusement être utilisée dans l'industrie alimentaire, par exemple dans les domaines de la confiserie, plus particulièrement dans celui du chewing-gum.

Dans le domaine du chewing-gum, comme il sera exemplifié ci-après, la quasi absence de fines particules dans la composition pulvérulente de cristaux de mannitol conforme à l'invention permet alors d'obtenir une meilleure coulabilité de la poudre, autorisant un remplacement total du talc dans le saupoudrage de la bande de chewing-gum, éliminant tout problème de toxicité potentiel du talc par la contamination éventuelle par de l'amiante, réduisant le passage dans l'air de la poudre, amoindrissant par là même la perte en poudre, et améliorant les conditions de travail des manipulateurs.

Toutefois, rien n'empêche de l'utiliser à une tout autre fin, comme par exemple dans les domaines :

- du baking (dans la garniture de pâtisseries tels les beignets (« doughnut »), plus particulièrement par son aptitude à l'écoulement, dans les systèmes de dosages et de mélange dans des applications mix de boulangerie/pâtisserie, boulangeries industrielles (facilité de dosage) ou pâtisserie industrielle.

- des fondants,

- des formes pharmaceutiques, tels que les sachets, gélules et comprimés,

- des préparations instantanées,

- des supports d'arômes,

- des supports d'édulcorants intenses,

- des céréales et céréales petit-déjeuner (en glaçage), et

- dans les sauces sans sucre ajouté.

L'invention sera encore mieux comprise à l'aide des exemples qui suivent, lesquels ne se veulent pas limitatifs et font seulement état de certains modes de réalisation et de certaines propriétés avantageuses de la composition pulvérulente de cristaux de mannitol selon l'invention.

**Exemple 1**

Un sirop de mannitol à 96% de mannitol a été cristallisé selon le brevet européen EP0202168.

La cristallisation est suivie d'une étape de séchage, une telle poudre étant commercialisée par la société demanderesse sous la dénomination MANNITOL P 60.

La poudre de mannitol cristallisé obtenue est introduite dans la trémie d'alimentation d'un sélecteur zig-zag dont le canal possède des angles de 120°, une largeur de 20 mm et une profondeur de 220mm. Il possède treize étages qui ont chacun une hauteur de 92 mm. L'alimentation s'effectue au niveau du 9eme étage. Différentes conduites de sélection sont menées afin d'obtenir des poudres de mannitol cristallisé définées (fraction grosses)

Pour cela, on adapte surtout le débit d'alimentation d'air primaire.

La vitesse de l'air ascendant définit en effet le diamètre de coupure du mélange initial.

C'est ainsi qu'en partant de la même poudre de cristaux de mannitol, en l'occurrence ici le MANNITOL 60, la mise en oeuvre d'un débit d'air primaire permet de faire varier la répartition granulométrique des poudres de mannitol definées.

Les conditions de mise en oeuvre sont présentées dans le tableau 1 suivant.

Tableau 1

| Poudre de mannitol selon l'invention | Débit poudre (kg/h) | Débit Air primaire (m³/h) | Débit Fractions (kg/h) | | Alimentation | | | Fraction grosse (µm) | | |
|---|---|---|---|---|---|---|---|---|---|---|
| | | | Débit fraction fines | Débit fraction grosse s | Diamètre arithmétique moyen (µm) D(4,3) | % en volume > 75 | % en volume > 250 | Diamètre arithmétique moyen (µm) D(4,3) | % en volume > 75 | % en volume > 250 |
| Produit « A » | 12 | 27 | 4.2 | 7.8 | 134 | 66.5 | 10.5 | 178 | 85.7 | 18.4 |
| Produit « B » | 12 | 36 | 9.6 | 2.4 | 134 | 66.5 | 10.5 | 228 | 95.1 | 32.5 |

### Exemple 2

Les poudres de l'exemple 1 ont été comparées aux poudres de mannitol cristallisées vendues par la demanderesse sous la dénomination Mannitol 35 et Mannitol 60 et à une poudre de mannitol cristallisée à partir d'un extrait du mannitol des algues (Mannitol vendu sous la dénomination mannitol cristallisé BRIGHTMOON®). Les résultats de ces mesures sont présentés au tableau 2.

Les poudres de l'exemple 1 ont été comparées à deux échantillons de mannitol cristallisées Mannitol 35 et Mannitol 60 (commercialisés par la demanderesse) ainsi qu'à une poudre de mannitol cristallisée à partir d'un extrait du mannitol des algues (Mannitol vendu sous la dénomination mannitol cristallisé BRIGHTMOON®). Les résultats de ces mesures sont présentés au tableau 2.

Parmi les échantillons testés, on remarque que seuls les échantillons A et B comportent à la fois une note d'écoulement traduisant de très bonnes caractéristiques d'écoulement et une compressibilité, reflet d'une grande stabilité au stockage. Ainsi, bien que comprenant une faible portion de particules inférieures à $75\mu m$ (10,7%), le Mannitol extrait d'algues présente une granulométrie moyenne supérieure à $300\mu m$ ($315\mu m$). Inversement, bien que comportant une granulométrie moyenne entre 100 et $300\mu m$, le Mannitol 60 présente un taux de particules inférieures à $75\mu m$ élevé de 33,5%.

Ces deux échantillons présentent un écoulement nettement inférieure aux échantillons A et B (le Mannitol extrait d'algues 52%, Mannitol 60 51,5%, A 62%, B 64,5%) mais néanmoins meilleur que l'échantillon Mannitol 35 (41,5%). Ceci démontre qu'une granulométrie comprise entre 100 et $300\mu m$ est une caractéristique en soi insuffisante pour obtenir une poudre cristalline de mannitol ayant un bon écoulement. Il en est de même d'une simple réduction de la proportion de particules de moins de $75\mu m$. Par contre, une forte proportion de particules ayant une granulométrie inférieure à $75\mu m$ et une granulométrie moyenne en dehors des bornes 100 et $300\mu m$ induit un très mauvais écoulement de la poudre (Mannitol 35 note d'écoulement 41,5%).

Concernant la caractéristique de compressibilité, le mannitol extrait d'algues présente une meilleure stabilité au stockage que le Mannitol 60 (respectivement une compressibilité de 28,1 et 34,8) mais celles-ci restent médiocres comparativement aux échantillons A et B (25,2 et 19,6). Ainsi, la stabilité au stockage dépend à la fois de la granulométrie moyenne de la poudre et de la richesse en particule de moins de $75\mu m$. Le Mannitol 35 qui présente à la fois une grande richesse en particules inférieures à $75\mu m$ et un diamètre moyen inférieure à $100\mu m$ comporte lui une très grande instabilité au stockage (compressibilité 43,4).

On remarque ainsi qu'il n'y a pas une adjonction des effets induits par ces deux caractéristiques de la poudre cristalline de mannitol (diamètre moyen de la poudre cristalline et taux de particules inférieure à $75\mu m$) mais réellement une coopération.

Tableau 2

| Echantillons | Mannitol 35 | Mannitol 60 | Produit « A » | Produit « B » | Mannitol extrait d'algues |
|---|---|---|---|---|---|
| Taux de particules inférieures à 75 $\mu m$ (%) | 65,9 | 33,5 | 14,3 | 4,9 | 10,7 |
| Taux de particules supérieures à 250$\mu m$ | 0,01 | 10,6 | 18,4 | 32,7 | 46,1 |
| Diamètre moyen ($\mu m$) | 67 | 135 | 178 | 228 | 315 |
| Note d'écoulement (sur 100) | 41,5 | 51,5 | 62 | 64,5 | 52 |
| Densité aérée | 0,450 | 0,535 | 0,595 | 0,615 | 0,615 |
| Densité tassée | 0,795 | 0,82 | 0,795 | 0,765 | 0,855 |
| Compressibilité | 43,4 | 34,8 | 25,2 | 19,6 | 28,1 |

### Exemple 3

Un test de mottage est réalisé en laboratoire. Ce test permet de simuler le mottage qui apparait dans les big-bags (sacs contenant de 500 à 1500Kg de poudre) de mannitol.

Une quantité de 1300 grammes de produit est placée dans un sachet de polyéthylène de 100 $\mu m$ d'épaisseur (dimension à plat de 32,4 cm sur 20,9 cm). Ce sachet est ensuite fermé hermétiquement après avoir chassé le maximum possible d'air occlus. Il est ensuite placé dans un cylindre perforé de 22 cm de hauteur et 13 cm de diamètre, percé de trous de 8mm de diamètre, disposés en quinconce avec une distance de 12 mm entre les centres des trous voisins. Un disque métallique de diamètre juste inférieur au cylindre est placé sur le sachet. Sur ce disque est posé un poids de 6.6 kg, soit l'équivalent d'une pression de 580kg/m$^2$, pression identique à celle que subit la poudre située au fond d'un big-bag.

L'ensemble est ensuite placé dans une enceinte climatique réglée de manière à lui faire subir 15 cycles de 6 heures (3 heures à une température de 15°C et une humidité relative de 85%, puis de 3 heures à une température de 30°C et une humidité relative de 85%).

A la fin de ces cycles, le sachet est retiré délicatement du cylindre et coupé. Une première observation de la poudre est effectuée. La totalité de la poudre est ensuite introduite dans un tonnelet de 5 litres (diamètre d'ouverture supérieur au diamètre du cylindre perforé), qui est mis en rotation une minute dans un mélangeur à retournement MIXOMAT A14 (FUSCHS/Suisse). La totalité de la poudre est ensuite versée sur un tamis dont les mailles ont des ouvertures carrées d'environ 8mm sur 8mm. On ne récupère ainsi que les mottes de produit de plus de 8mm environ de diamètre, dont le poids total est mesuré. Le taux de produit motté est calculé en divisant le poids de ces mottes par le poids initial de mannitol mis en oeuvre (1300 grammes).

Tableau 3

| Echantillons | Mannitol 35 | Mannitol 60 | A | B | Mannitol extrait d'algues |
|---|---|---|---|---|---|
| Aspect de la poudre | Bloc dur | Bloc friable | Bloc très friable | Poudre fluide | Bloc très friable |
| Taux de produit motté | 24% | 18% | 8% | 0% | 10% |

Les Mannitol 35 et Mannitol 60 ont un taux de produit motté élevé, ce qui indique que la poudre située au fond des big-bags va acquérir de la cohésion très rapidement après remplissage et que ces big-bags deviendront très difficiles à vider. Ce conditionnement est donc à déconseiller pour ces deux échantillons. Pour l'échantillon A et le mannitol extrait d'algues qui présentent un taux de mottage de 8%-10%, ce conditionnement est donc envisageable, mais le stockage devra être limité dans le temps. Pour l'échantillon B, le remplissage, le stockage et le vidage des big-bags ne présentera aucune difficulté : il peut sans souci être commercialisé dans ce type d'équipement.

**Exemple 4**

Une production industrielle de chewing-gum est réalisée sur une ligne de production de marque TOGUM (BOSCH-TOGUM).

Cette production est réalisée avec une formule standard de chewin-gum « sans sucre » .

- Gomme base : 32%

- Sorbitol poudre NEOSORB® P60W : 49%

- Mannitol 60 : 7%

- Sirop de maltitol LYCASIN® 80/55HDS : 9%

- Glycérine: 0,2%

- Aspartame : 0,2%

- Arôme menthe liquide : 2,1%

- Arôme menthe poudre : 0,5%

L'étape de mélange est réalisée dans un pétrin bras en z TOGUM GT120, d'une capacité d'environ 60kg. Le mélange est réalisé en continu.

A t=0, on introduit dans le pétrin la gomme base préalablement chauffée une nuit à 50°C et la moitié du sorbitol poudre. A t=3 min, on introduit le mannitol, à t=5 min le sirop de maltitol, à t=7 min la moitié du sorbitol et l'aspartame, à t= 11min la glycérine, à t=12 min les arômes. A t = 16 min, le mélange est stoppé et la pâte déchargée. La température de la pâte est alors d'environ 55°C. On divise celle-ci en pains d'environ 2 kg qui sont stockés 1 heure à 20°C et 50% d'humidité relative, ce qui amènera la température de la pâte à 47°C avant extrusion.

L'étape d'extrusion est réalisée sur un équipement TOGUM TO-E82, avec le corps de l'extrudeuse chauffé à 40°C et la tête à 45°C.

L'étape de saupoudrage et l'étape de laminage sont réalisées sur un laminoir TOGUM TO-W191. Il est équipé en premier lieu de deux postes de saupoudrage, un situé sur le dessus de la bande de chewing-gum extrudée et un au dessus d'une bande transporteuse située en dessous de la bande de chewing-gum, et dont le rôle est d'apporter la poudre de saupoudrage sur la face interne du chewing-gum. Ainsi, la bande de chewing-gum est saupoudrée sur les deux faces avant le premier poste de laminage. Il est équipé ensuite de 4 paire de rouleaux de laminage, avec, situé entre la seconde et la troisième paires, un système de dépoussiérage constitué d'une paire de brosses, l'une située sur le dessous et l'autre sur le dessus de la bande de chewing-gum. Ce système sert à enlever l'excès de poudre sur les deux faces de la bande de chewing-gum. Il est équipé enfin de deux paires de rouleaux pour le formage et la découpe afin de conférer au chewing-gum la forme finale recherchée, dans le cas présent, les coussinets.

Les poudres de mannitol de l'exemple 2 ont été testées en saupoudrage. La poudre de saupoudrage était constituée uniquement de ces poudres de mannitol : aucun talc n'a été ajouté.

Les observations effectuées ont été : la facilité à obtenir l'écoulement de la poudre de l'équipement de saupoudrage, la maîtrise de la quantité de poudre déposée par rapport à la quantité souhaitée, la quantité de poudre perdue, la formation de poussières en suspension dans l'air, et l'aspect du chewing-gum après dépoussiérage. De plus, les chewing-gums ont été testés par un jury de 15 panelistes pour déterminer si l'augmentation de la taille des particules de la poudre de saupoudrage confère au chewing-gum une texture sableuse. Les essais sont effectués selon la norme AFNOR V 09-014 (avril 1982) sur les échantillons A à Z par groupe de 5 ou 6 échantillons par essai. Les 5 ou 6 échantillons ont été présentés simultanément, en imposant un ordre de dégustation différent pour chaque membre du jury. Le descripteur imposé à savoir le caractère sableux en bouche, est évalué sur une échelle à 6 points graduée de la façon suivante : absence, très faible, faible, net, prononcé, très prononcé. L'analyse de variance (ANOVA de Friedman) discrimine les échantillons sur leurs caractères sableux ($p \ll 0.05$). Les valeurs obtenues sont reprises dans le tableau 4.

Tableau 4

| Echantillons | Mannitol 35 | Mannitol 60 | Echantillon A | Echantillon B | Mannitol extrait d'algues |
|---|---|---|---|---|---|
| Ecoulement de la poudre | Mauvais | Mauvais | Moyen | Bon | Mauvais |
| Maîtrise de la quantité de poudre saupoudrée | Mauvaise | Passable | Plutôt bonne | Bonne | Mauvaise |
| Quantité de poudre perdue | Très élevée | Elevée | Moyenne | Faible | Moyenne |
| Particules en suspension dans l'air | Beaucoup | Beaucoup | Peu | Très peu | Très peu |
| Aspect du chewing-gum après dépoussiérage | Conforme | Conforme | Conforme | Conforme | Conforme |
| Sensation sableuse à la dégustation en bouche | Absence | Absence | Absence | Absence | Net |

Les Mannitol 35, Mannitol 60 et le mannitol extrait d'algues, présentent un mauvais écoulement, ce qui rend difficile le réglage de l'équipement de saupoudrage. La quantité déposée est donc difficile à maîtriser. En conséquence, le taux de perte est important. Or, alors que les Mannitols 35 et 60 comportent un taux de particules inférieures à $75\mu m$ supérieur à 28%, le mannitol extrait d'algues comporte lui, un taux particulièrement faible de ces mêmes particules. Les échantillons A et B, présentant moins de 25% de particules inférieures à $75\mu m$ et un diamètre moyen de particules de 178 et $228\mu m$ possèdent un écoulement qui permet de maîtriser la quantité de poudre déposée et de limiter les pertes.

Par ailleurs, de par la présence de fines dans les Mannitol 35 et Mannitol 60, le taux de particules en suspension dans l'air est élevé, par opposition aux échantillons A et B ou au Mannitol extrait d'algues pour lesquels on observe une faible densité de particules dans l'air. La faible quantité de particules en suspension est un avantage pour la propreté des locaux et la santé des opérateurs.

Le diamètre moyen des poudres A et B permet à la fois de favoriser l'écoulement des poudres, de réduire le taux de particules dans l'air lors de la manipulation de ces poudres tout en n'ayant pas de conséquences négatives sur les qualités organoleptiques du chewing-gum obtenu. En effet, alors que l'usage du mannitol extrait d'algues induit une sensation sableuse en bouche, les échantillons A et B tout en permettant une bonne gestion de dépôt de poudre de saupoudrage avec un taux réduit de poudre en suspension dans l'air ainsi qu'un saupoudrage uniforme des bandes de chewing-gum après dépoussiérage n'entraine aucune sensation sableuse en bouche.

**EP 2 473 465 B1**

**Revendications**

1. Composition pulvérulente de cristaux de mannitol obtenue par (i) cristallisation de mannitol dans un solvant suivie (ii) d'une étape de séparation des cristaux de la suspension de cristaux obtenue, (iii) d'une étape de séchage des cristaux et (iv) d'une étape de sélection, ladite composition **caractérisée en ce qu'**elle présente une répartition granulométrique en volume, déterminée par granulométrie laser, présentant :

   - de 72 à 99,9%, de préférence de 75 à 99%, plus préférentiellement de 80 à 98%, de particules de granulométrie supérieure à 75µm,
   - de 0,1 à 60%, de préférence de 1 à 55%, plus préférentiellement de 2 à 40%, de particules supérieures à 250µm,
   - un diamètre moyen de 100 à 300µm, de préférence de 120 à 270µm.

2. Composition pulvérulente selon la revendication 1, **caractérisée en ce qu'**elle présente une note d'écoulement supérieur ou égale à 55, préférentiellement située entre 60 et 90.

3. Composition pulvérulente selon l'une ou l'autre des revendications 1 ou 2, **caractérisée en ce qu'**elle présente une richesse en mannitol de 96 à 100% en poids, de préférence entre 97% et 99,9% en poids.

4. Composition pulvérulente selon l'une quelconque des revendications 1 à 3, **caractérisée en ce qu'**elle présente une compressibilité comprise entre 30 et 15%, de préférence entre 27 et 12.

5. Composition pulvérulente selon l'une quelconque des revendications 1 à 4, **caractérisée en ce qu'**elle présente une densité aérée supérieure à 0,480 g/ml, de préférence comprise entre 0,540 et 0,700 g/ml, et une densité tassée comprise entre 0,700 et 0,860 g/ml, de préférence comprise entre 0,725 et 0,830 g/ml.

6. Procédé d'obtention d'une composition pulvérulente de cristaux de mannitol comprenant de 72 à 99,9%, de préférence de 75 à 99%, plus préférentiellement de 80 à 98%, et plus préférentiellement encore de 85 à 97% de particules de granulométrie supérieure à 75µm, ledit procédé comprenant :

   a) une étape de cristallisation de mannitol dans un solvant, préférentiellement dans l'eau
   b) une étape de séparation des cristaux de la suspension de cristaux obtenue
   c) une étape de séchage des cristaux
   d) une étape de sélection des cristaux, et
   e) une étape de récupération de la composition pulvérulente.

7. Procédé selon la revendication 6, **caractérisé en ce que** l'étape de sélection b) s'effectue par tamisage ou sélecteur pneumatique.

8. Procédé selon la revendication 7, **caractérisé en ce que** l'étape de sélection b) s'effectue par un sélecteur zig-zag.

9. Procédé selon la revendication 8, **caractérisé en ce que** l'étape de sélection b) s'effectue par un sélecteur zig-zag comprenant plusieurs étages, préférentiellement 13 étages.

10. Procédé selon l'une ou l'autre des revendications 8 et 9, **caractérisé en ce que** l'étape de sélection b) des particules s'effectue par un sélecteur zig-zag comprenant un seul canal, ce canal présentant préférentiellement une largeur de 20 mm et une profondeur de 220mm.

11. Procédé selon l'une quelconque des revendications 8 à 10 **caractérisé en ce que** l'étape de sélection b) comprend les étapes suivantes :

   - b.1) alimentation d'un sélecteur Zig-Zag avec une composition pulvérulente de cristaux de mannitol présentant un diamètre moyen arithmétique compris entre 80 et 145 µm,

   b.2) réglage du débit d'air primaire de manière à récupérer une fraction de composition pulvérulente de cristaux de mannitol présentant un diamètre moyen de 100 à 300µm et une répartition granulométrie en volume, déterminée par granulométrie laser, de 0,1 à 60% de particules supérieures à 250µm.

**Patentansprüche**

1. Pulverförmige Zusammensetzung aus Mannitolkristallen, erhalten durch (i) Kristallisation von Mannitol aus einem Lösungsmittel, gefolgt von (ii) einem Trennungsschritt der Kristalle aus der erhaltenen Kristallsuspension, (iii) einem Trocknungsschritt der Kristalle und (iv) einem Auswahlschritt, wobei die Zusammensetzung **dadurch gekennzeichnet ist, dass** sie eine mittels Partikelgrößenlaser bestimmte, volumenmäßige Partikelgrößenverteilung aufweist, welche:

   - 72 bis 99,9 %, bevorzugt 75 bis 99%, stärker bevorzugt 80 bis 98%, Partikel mit einer Partikelgröße von mehr als 75 $\mu$m,
   - 0,1 bis 60 %, bevorzugt 1 bis 55%, stärker bevorzugt 2 bis 40%, Partikel mit mehr als 250 $\mu$m,
   - einen mittleren Durchmesser von 100 bis 300 $\mu$m, bevorzugt 120 bis 270 $\mu$m

   aufweist.

2. Pulverförmige Zusammensetzung nach Anspruch 1, **dadurch gekennzeichnet, dass** sie einen Strömungswert von mehr als oder gleich 55, bevorzugt zwischen 60 und 90, aufweist.

3. Pulverförmige Zusammensetzung nach einem der Ansprüche 1 oder 2, **dadurch gekennzeichnet, dass** sie eine Mannitolanteil von 96 bis 100 Gew.-%, bevorzugt zwischen 97 Gew.-% und 99,9 Gew.-% aufweist.

4. Pulverförmige Zusammensetzung nach einem der Ansprüche 1 bis 3, **dadurch gekennzeichnet, dass** sie eine Kompressibilität im Bereich von 30 bis 15 %, bevorzugt von 27 bis 12, aufweist.

5. Pulverförmige Zusammensetzung nach einem der Ansprüche 1 bis 4, **dadurch gekennzeichnet, dass** sie eine Schüttdichte von mehr als 0,480 g/ml, bevorzugt im Bereich von 0,540 bis 0,700 g/ml, und eine Packdichte im Bereich von 0,700 bis 0,860 g/ml, bevorzugt im Bereich von 0,725 und 0,830 g/ml aufweist.

6. Verfahren zur Herstellung einer pulverförmigen Zusammensetzung aus Mannitolkristallen, welche 72 bis 99,9 %, bevorzugt 75 bis 99 %, stärker bevorzugt 80 bis 98 %, und noch stärker bevorzugt 85 bis 97 % Partikel mit einer Partikelgröße von mehr als 75 $\mu$m aufweist, wobei das Verfahren umfasst:

   a) einen Kristallisationsschritt von Mannitol aus einem Lösungsmittel, bevorzugt aus Wasser
   b) einem Trennungsschritt der Kristalle aus der erhaltenen Kristallsuspension,
   c) einem Trocknungsschritt der Kristalle
   d) einem Auswahlschritt der Kristalle, und
   e) einem Rückgewinnungsschritt der pulverförmigen Zusammensetzung.

7. Verfahren nach Anspruch 6, **dadurch gekennzeichnet, dass** der Auswahlschritt b) durch Sieben oder eine pneumatische Auswahlvorrichtung erfolgt.

8. Verfahren nach Anspruch 7, **dadurch gekennzeichnet, dass** der Auswahlschritt b) durch eine Zick-Zack Auswahlvorrichtung erfolgt.

9. Verfahren nach Anspruch 8, **dadurch gekennzeichnet, dass** der Auswahlschritt b) durch eine mehrere Stufen, bevorzugt 13 Stufen, umfassende Zick-Zack Auswahlvorrichtung erfolgt.

10. Verfahren nach einem der Ansprüche 8 oder 9, **dadurch gekennzeichnet, dass** der Auswahlschritt b) der Partikel durch eine Zick-Zack Auswahlvorrichtung erfolgt, welche einen einzigen Kanal umfasst, wobei der Kanal bevorzugt eine Breite von 20 mm und eine Tiefe von 220 mm aufweist.

11. Verfahren nach einem der Ansprüche 8 bis 10, **dadurch gekennzeichnet, dass** der Auswahlschritt b) die folgenden Schritte umfasst:

   - b.1) Zuführen einer pulverförmigen Zusammensetzung aus Mannitolkristallen mit einem arithmetischen, mittleren Durchmesser im Bereich von 80 bis 145 $\mu$m in eine Zick-Zack Auswahlvorrichtung,
   - b.2) Einstellen des primären Luftstroms auf eine Weise, dass ein Teil der pulverförmigen Zusammensetzung aus Mannitolkristallen mit einem arithmetischen, mittleren Durchmesser im Bereich von 100 bis 300 $\mu$m und

einer mittels Partikelgrößenlaser bestimmte, volumenmäßige Partikelgrößenverteilung von 0,1 bis 60 % Partikel mit mehr als 250 $\mu$m zurückgewonnen wird.

**Claims**

1.  A pulverulent composition formed of mannitol crystals which is obtained by (i) crystallization of mannitol from a solvent, followed (ii) by a step of separation of the crystals from the suspension of crystals which is obtained, (iii) by a step of drying the crystals and (iv) by a step of selection, wherein the particle size distribution by volume, determined by laser particle size analysis, exhibits:

    - from 72 to 99.9%, preferably from 75 to 99% and more preferably from 80 to 98% of particles with a particle size of greater than 75 $\mu$m,
    - from 0.1 to 60%, preferably from 1 to 55% and more preferably from 2 to 40% of particles of greater than 250 $\mu$m,
    - a mean diameter of 100 to 300 $\mu$m, preferably of 120 to 270 $\mu$m.

2.  The pulverulent composition as claimed in claim 1, wherein the pulverulent composition has a flow grade of greater than or equal to 55, preferably situated between 60 and 90.

3.  The pulverulent composition as claimed in either of claims 1 and 2, wherein the pulverulent composition has a mannitol richness is from 96 to 100% by weight, preferably between 97 and 99.9% by weight.

4.  The pulverulent composition as claimed in any one of claims 1 to 3, wherein the pulverulent composition has a compressibility of between 30 and 15%, preferably between 27 and 12%.

5.  The pulverulent composition as claimed in any one of claims 1 to 4, wherein the pulverulent composition has an aerated density of greater than 0.480 g/ml, preferably of between 0.540 and 0.700 g/ml, and a packed density of between 0.700 and 0.860 g/ml, preferably of between 0.725 and 0.830 g/ml.

6.  A process for producing a pulverulent composition formed of mannitol crystals comprising from 72 to 99.9%, preferably from 75 to 99%, more preferably from 80 to 98% and more preferably still from 85 to 97% of particles with a particle size of greater than 75 $\mu$m, said process comprising:

    a) a step of crystallization of mannitol from a solvent, preferably from water,
    b) a step of separation of the crystals from the suspension of crystals which is obtained,
    c) a step of drying the crystals,
    d) a step of selection of the crystals, and
    e) a step of recovery of the pulverulent composition.

7.  The process as claimed in claim 6, wherein the step d) of selection is carried out by sieving or with an air classifier.

8.  The process as claimed in claim 7, wherein the step d) of selection is carried out with a zigzag classifier.

9.  The process as claimed in claim 8, wherein the step d) of selection is carried out with a zigzag classifier comprising several stages, preferably 13 stages.

10. The process as claimed in either of claims 8 and 9, wherein the step d) of selection of the particles is carried out by a zigzag classifier comprising just one channel, this channel preferably exhibiting a width of 20 mm and a depth of 220 mm.

11. The process as claimed in any one of claims 8 to 10, wherein the step d) of selection comprises the following steps:

    - d.1) feeding a zigzag classifier with a pulverulent composition formed of mannitol crystals exhibiting a mean arithmetic diameter of between 80 and 145 $\mu$m,
    - d.2) regulating the primary air flow rate so as to recover a fraction of pulverulent composition formed of mannitol crystals exhibiting a mean diameter of from 100 to 300 $\mu$m and a particle size distribution by volume, determined by laser particle size analysis, of from 0.1 to 60% of particles of greater than 250 $\mu$m.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0645096 A1
- EP 0645096 A
- EP 1138661 A1
- EP 1138661 A
- EP 0202168 A
- US 1861248 A